# EUROPÄISCHE PATENTSCHRIFT

(11) **EP 0 987 243 B1**
(45) Veröffentlichungstag und Bekanntmachung des Hinweises auf die Patenterteilung: **04.12.2002**
(21) Anmeldenummer: 99115272.9
(22) Anmeldetag: 31.07.1999
(51) Int. Cl.: C07C 29/16, C07C 29/141, C07C 31/125

(54) **Verfahren zur Herstellung von höheren Oxo-Alkoholen aus Olefingemischen durch zweistufige Hydroformylierung**
Process for the preparation of higher oxo-alcohols from olefin mixtures by two-step hydroformylation
Procédé pour la préparation d'oxo alcools supérieurs à partir de mélanges d'oléfines par hydroformylation en deux étapes

(30) Priorität: 16.09.1998 DE 19842368
(43) Veröffentlichungstag der Anmeldung: 22.03.2000
(73) Patentinhaber: Oxeno Olefinchemie GmbH, 45764 Marl (DE)
(72) Erfinder: Kaizik, Alfred, Dr., 45772 Marl (DE); Scholz, Bernhard, Dr., 45768 Marl (DE); Tötsch, Walter, Dr., 45770 Marl (DE); Büschken, Wilfried, Dr., 45721 Haltern (DE); Nierlich, Franz, Dr., 45768 Marl (DE); Röttger, Dirk, Dr., 45657 Recklinghausen (DE); Wiese, Klaus-Diether, Dr., 45721 Haltern (DE)

(56) Entgegenhaltungen:
- EP-A- 0 183 545
- EP-A- 0 326 674
- DE-A- 1 935 900
- DE-A- 3 232 557
- US-A- 4 533 755

## Beschreibung

Die Erfindung betrifft ein Verfahren zur Herstellung höherer Oxo-Alkohole durch zweistufige Hydroformylierung von Olefingemischen, des eine selektive Hydrierung der Hydroformylierungsgemische einschließt.

### Stand der Technik

Höhere Alkohole, insbesondere solche mit 6 bis 25 C-Atomen, können bekanntlich durch katalytische Hydroformylierung (oder Oxo-Reaktion) der um ein C-Atom ärmeren Olefine und anschließende katalytische Hydrierung der Aldehyd- und Alkohol-haltigen Reaktionsgemische hergestellt werden. Sie werden vorwiegend als Edukte für die Herstellung von Weichmachern oder Detergentien verwendet.

Die Art des Katalysatorsystems und die optimalen Reaktionsbedingungen für die Hydroformylierung sind von der Reaktivität des eingesetzten Olefins abhängig. Die Abhängigkeit der Reaktivität der Olefine von ihrer Struktur ist z.B. von J.Falbe. New Syntheses with Carbon Monoxide, Springer-Verlag, Berlin, Heidelberg, New York, 1980. Seite 95 ff., beschrieben. Die unterschiedliche Reaktivität speziell der isomeren Octene ist ebenfalls bekannt (B.L.Haymore. A. van Hasselz. R.Beck. Annals of the New York Acad.Sci., 415 (1983), Seiten 159-175).

Technische Olefingemische, die als Edukte für die Oxo-Synthese verwendet werden, enthalten Olefinisomere der verschiedensten Strukturen mit unterschiedlichen Verzweigungsgraden, unterschiedlicher Lage der Doppelbindung im Molekül und gegebenenfalls auch unterschiedlichen C-Zahlen. Dies gilt besonders für Olefingemische, die durch Di-, Tri- oder weitergehende Oligomerisierung von C₂-C₅-Olefinen oder anderen leicht zugänglichen höheren Olefinen bzw. durch Cooligomerisierung der genannten Olefine entstanden sind. Als Beispiele für typische isomere Olefingemische, die durch Rhodium-katalysierte oder vorzugsweise durch Kobalt-katalysierte Hydroformylierung zu den entsprechenden Aldehyd- und Alkohol-Gemischen umgesetzt werden können, seien Tri- und Tetrapropene sowie Di-, Tri- und Tetrabutene genannt.

Die Geschwindigkeit der Hydroformylierungsreaktion nimmt mit steigender C-Zahl sowie mit dem Verzweigungsgrad ab. Die Reaktionsgeschwindigkeit von linearen Olefinen kann um den Faktor 5 bis 10 größer sein als die der verzweigten Isomeren. Auch die Lage der Doppelbindung im Olefinmolekül beeinflußt die Reaktivität. Olefine mit endständiger Doppelbindung reagieren deutlich schneller als Isomere mit der Doppelbindung im Inneren des Moleküls. Wegen der unterschiedlichen Reaktivität der Olefinisomeren bedarf es relativ langer Reaktionszeiten. wenn man einen möglichst weitgehenden Umsatz der Olefine erreichen will. Dadurch wird jedoch die Produktausbeute infolge unerwünschter Neben- und Folgereaktionen vermindert. Dasselbe tritt ein, wenn man die Reaktionszeiten durch höhere Reaktionstemperaturen abzukürzen trachtet. Vor allem wegen der unterschiedlichen Reaktivität der Isomeren ist es schwierig, bei der Hydroformylierung von Olefingemischen hohe Umsätze und gleichzeitig hohe Selektivitäten zu erzielen. Das gilt insbesondere für einstufige Hydroformylierungen.

Nach DE 32 32 557 A1 werden Alkohole durch eine zweistufige Hydroformylierung von Monoolefinen mit 3 bis 20 Kohlenstoffatomen hergestellt. In der ersten Reaktionsstufe werden die Olefine unter Verwendung eines Kobaltkatalysators mit Umwandlungsgraden von 50 bis 90% zum Aldehyd umgesetzt, wobei die Bildung von Alkoholen unterdrückt wird. Danach wird der Kobaltkatalysator vom Reaktionsgemisch abgetrennt und dieses in einer zweiten Stufe unter Verwendung eines Kobaltorganophosphin-Komplexes als Katalysator erneut hydroformyliert. Gleichzeitig wird der in der ersten Stufe gebildete Aldehyd zum Alkohol hydriert. Nachteilig ist bei diesem Verfahren, daß besonders in der zweiten Hydroformylierungsstufe ein erheblicher Teil der Olefine hydriert statt hydroformyliert wird.

DE 19 35 900 offenbart die Hydrierung von Aldehyden und Ketonen in kohlenmonoxidreichen Gasströmen.

### Kurzbeschreibung der Erfindung

Eine Aufgabe der Erfindung besteht darin, ein Verfahren zur Herstellung von höheren Oxo-Alkoholen aus den entsprechenden Olefingemischen bereitzustellen, das hohe Umsätze mit hohen Selektivitäten verbindet und sich zudem durch hohe Raum-Zeit-Ausbeuten auszeichnet.

Gegenstand der Erfindung ist dementsprechend ein Verfahren zur Herstellung von höheren Oxo-Alkoholen aus Gemischen isomerer Olefine mit 5 bis 24 Kohlenstoffatomen durch zweistufige Hydroformylierung in Gegenwart eines Kobalt- oder Rhodium-Katalysators bei erhöhter Temperatur und unter erhöhtem Druck, bei dem man das Reaktionsgemisch der ersten Hydroformylierungsstufe selektiv hydriert, das Hydrierungsgemisch in einer Destillation in rohen Alkohol und überwiegend aus Olefinen bestehende Leichtsieder trennt, diese in die zweite Hydroformylierungsstufe führt, das Reaktionsgemisch der zweiten Hydroformylierungsstufe wiederum selektiv hydriert, das Hydrierungsgemisch in einer Destillation wiederum in rohen Alkohol und Leichtsieder trennt. den rohen Alkohol durch Destillation auf reinen Alkohol aufarbeitet und zumindest einen Teil der Leichtsieder zur Ausschleusung gesättigter Kohlenwasserstoffe abzieht.

Die selektive Hydrierung erfolgt bei erhöhter Temperatur und erhöhtem Druck an einem Trägerkatalysator, der als aktive Komponenten Kupfer, Nickel und Chrom enthält.

Vorteilhaft werden die entspannten Reaktionsgemische aus beiden Hydroformylierungsstufen vor der selektiven Hydrierung vom Hydroformylierungskatalysator befreit.

### Beschreibung der Erfindung

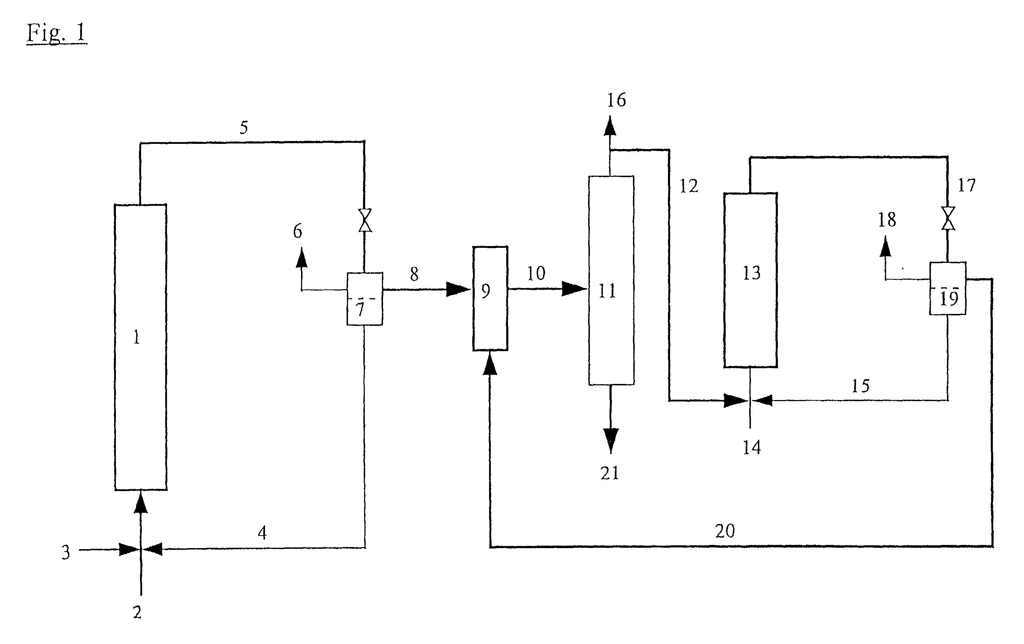

Das erfindungsgemäBe Verfahren kann diskontinuierlich oder vorteilhaft kontinuierlich durchgeführt werden. Für die kontinuierliche Durchführung sind verschiedene Verfahrensvarianten möglich. In der Figur 1 ist als Beispiel das Blockschema einer Anlage dargestellt, in der das Verfahren kontinuierlich durchgeführt werden kann. In den ersten Hydroformylierungsreaktor 1 werden das Olefingemisch 2, Synthesegas (Kohlenmonoxid und Wasserstoff) 3 sowie Katalysator 4 eingeführt. Das Hydroformylierungsgemisch 5 wird entspannt, das Entspannungsgas 6 (nicht verbrauchtes Synthesegas) wird abgezogen und das entspannte Hydroformylierungsgemisch 5 in der ersten Katalysatorabtrennung 7 vom Katalysator 4 befreit, der, gegebenenfalls nach Ergänzung durch frischen Katalysator, in den ersten Hydroformylierungsreaktor 1 zurückgeführt wird. Das vom Katalysator befreite Hydroformylierungsgemisch 8 wird in die selektive Hydrierung 9 geleitet, in der die Aldehyde und im Gemisch enthaltene Nebenprodukte, wie Acetale der Aldehyde und Ester der Alkohole, insbesondere deren Formiate, zu den Alkoholen hydriert werden. Aus dem Hydrierungsgemisch 10 werden in der Destillation 11 die Leichtsieder 12 abgetrennt, die weit überwiegend aus nicht umgesetzten isomeren Olefinen bestehen und in den zweiten Hydroformylierungsreaktor 13 geführt werden, in den man auch Synthesegas 14 und Katalysator 15 einführt. Ein Teil der Leichtsieder 12 wird als restliche Leichtsieder 16 ausgeschleust. Das Hydroformylierungsgemisch 17 aus dem zweiten Hydroformylierungsreaktor 13 wird wiederum entspannt, und Entspannungsgas 18 wird abgezogen. Das entspannte Hydroformylierungsgemisch 17 wird in der zweiten Katalysatorabtrennung 19 vom Katalysator 15 befreit, der, wiederum gegebenenfalls nach Ergänzung, in den zweiten Hydroformylierungsreaktor 13 zurückgeführt wird, und als entkatalysiertes Hydroformylierungsgemisch 20 in die selektive Hydrierung 9 überführt. Dort wird es zusammen mit dem vom Katalysator befreiten Hydroformylierungsgemisch 8 aus dem ersten Hydroformylierungsreaktor 1 selektiv hydriert. Der aus der Destillation 11 abgezogene rohe Alkohol 21 wird in einer nicht dargestellten weiteren Destillation auf reinen Alkohol aufgearbeitet.

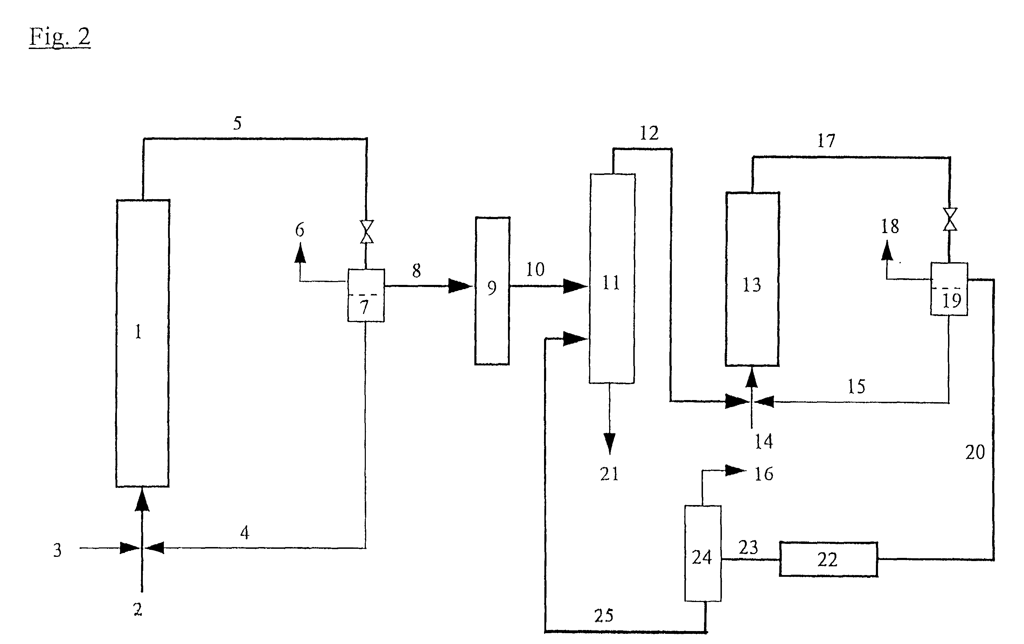

Das Blockschema einer alternativen zweiten kontinuierlich arbeitenden Verfahrensvariante zur Durchführung des Verfahrens nach der Erfindung ist in der Figur 2 dargestellt. In den ersten Hydroformylierungsreaktor 1 werden das Olefingemisch 2, Synthesegas 3 sowie Katalysator 4 eingeführt. Das Hydroformylierungsgemisch 5 wird entspannt, das Entspannungsgas 6 wird abgezogen, und das entspannte Hydroformylierungsgemisch in der ersten Katalysatorabtrennung 7 vom Katalysator 4 befreit, der, gegebenenfalls nach Ergänzung durch frischen Katalysator, in den ersten Hydroformylierungsreaktor 1 zurückgeführt wird. Das vom Katalysator befreite Hydroformylierungsgemisch 8 wird in die erste selektive Hydrierung 9 geleitet, in der die Aldehyde sowie die als Nebenprodukte enthaltenen Acetale und Ester, insbesondere die Formiate der Alkohole, zu den Alkoholen hydriert werden. Aus dem ersten Hydrierungsgemisch 10 werden in der ersten Destillation 11 die Leichtsieder 12 abgetrennt, die weit überwiegend aus nicht umgesetzten isomeren Olefinen bestehen und in den zweiten Hydroformylierungsreaktor 13 geführt werden, in den man auch Synthesegas 14 und Katalysator 15 einführt. Das Hydroformylierungsgemisch 17 aus dem zweiten Hydroformylierungsreaktor 13 wird wiederum entspannt, und Entspannungsgas 18 wird abgezogen. Das entspannte Hydroformylierungsgemisch 17 wird in der zweiten Katalysatorabtrennung 19 vom Katalysator 15 befreit, der, wiederum gegebenenfalls nach Ergänzung mit frischem Katalysator, in den zweiten Hydroformylierungsreaktor 13 zurückgeführt wird, und als entkatalysiertes Hydroformylierungsgemisch 20 in eine zweite, vergleichsweise kleine selektive Hydrierung 22 geleitet. Das zweite Hydrierungsgemisch 23 wird in der zweiten, vergleichsweise kleinen Destillation 24 in paraffinreiche Leichtsieder 16, die abgezogen werden, und rohen Alkohol 25 zerlegt, der in die erste Destillation 11 geleitet und dort zusammen mit dem ersten Hydrierungsgemisch 10 destilliert wird. Der rohe Alkohol 21 wird wiederum in einer nicht dargestellten weiteren Destillation auf reinen Alkohol aufgearbeitet.

Ein wesentlicher Unterschied der beiden Verfahrensweisen besteht darin, daß nach Figur 1 nur eine selektive Hydrierung 9 vorgesehen wird, in denen die beiden entkatalysierten Hydroformylierungsgemische 8 und 20 hydriert werden, und nur eine Destillation 11, in der das Hydrierungsgemisch 10 getrennt wird, während nach Figur 2 das zweite Hydroformylierungsgemisch 20 in einer zweiten selektiven Hydrierung 22 hydriert und das Hydrierungsgemisch in einer zweiten Destillation 24 getrennt wird. Die Variante nach Figur 2 ist in der Investition teurer, weil zusätzliche, allerdings vergleichsweise kleine Apparate benötigt werden. Dafür wird das Olefin weitergehend ausgenutzt. denn die Leichtsieder 16 nach Figur 2 sind der Menge nach geringer als die Leichtsieder 16 nach Figur 1 und zudem weitgehend frei von Olefinen. während die Leichtsieder 16 nach Figur 1 noch erhebliche Mengen an Olefinen enthalten.

### Hydroformylierung

Die Edukte für die Hydroformylierung sind Gemische von Monoolefinen mit 5 bis 24 Kohlenstoffatomen und end- oder mittelständiger C-C-Doppelbindung, wie 1- oder 2-Penten, 2-Methyl-1-buten, 1-, 2- oder 3-Hexen, das bei der Dimerisierung von Propen anfallende isomere C₆-Olefingemisch (Dipropen), 1-Hepten, 2- oder 3-Methyl-1-hexen, 1-Octen, das bei der Dimerisierung von Butenen anfallende isomere C₈-Olefingemisch (Dibuten), 1-Hepten, 1-Nonen, 2- oder 3-Methyl-1-octen, das bei der Trimerisierung von 1-Propen anfallende isomere C₉-Olefingemisch (Tripropen), 1-, 2- oder 3-Decen, 2-Ethyl-1-octen, 1-Dodecen, das bei der Tetramerisierung von Propen oder der Trimerisierung von Butenen anfallende isomere C₁₂-Olefingemisch (Tetrapropen oder Tributen), 1-Tetradecen, 1- oder 2-Hexadecen, bei der Tetramerisierung von Butenen anfallende C₁₆-Olefingemische (Tetrabuten) sowie durch Cooligomerisierung von Olefinen mit unterschiedlichen C-Zahlen (bevorzugt 2 bis 4) hergestellte Olefingemische, gegebenenfalls nach destillativer Trennung in Fraktionen mit gleicher oder ähnlicher C-Zahl. Bevorzugte Edukte sind C₈-, C₉-, C₁₂- oder C₁₆-Olefingemische.

Die Erfindung liegt nicht in der Art oder den Bedingungen der Hydroformylierung in den beiden Stufen. Die Olefine werden vielmehr in an sich bekannter Weise hydroformyliert. Man arbeitet also mit Rhodium- oder vorzugsweise in beiden Stufen mit Kobaltkatalysatoren sowie mit oder ohne Komplex-stabilisierenden Zusätzen, wie organischen Phosphinen oder Phosphiten. Die Temperaturen und die Drücke können, je nach Katalysator und Olefingemisch, in weiten Grenzen variieren. Da in der ersten Stufe bevorzugt die reaktiveren Olefine reagieren, stellt man in der zweiten Hydroformylierung zweckmäßig energischere Reaktionsbedingungen hinsichtlich Temperatur, Katalysatormenge usw. ein. Für ein gegebenes Olefingemisch lassen sich die optimalen Bedingungen für die beiden Hydroformylierungsstufen durch Versuche unschwer ermitteln. Eine Beschreibung der Hydroformylierung von Olefinen findet sich z.B. bei J.Falbe, New Syntheses with Carbon Monoxide, Springer-Verlag. Heidelberg-New York, 1980, Seiten 99ff, sowie bei Kirk-Othmer, Encyclopedia of Chemical Technology, Band 17, 4. Auflage, John Wiley & Sons, Seiten 902-919 (1996).

Im allgemeinen führt man die Hydroformylierung in der ersten Stufe so, daß 50 bis 90%, vorzugsweise 60 bis 85% des zugeführten Olefingemisches umgesetzt werden. Natürlich müssen die Hydroformylierungsreaktoren und die sonstigen Apparate entsprechend ausgelegt sein.

Der Umsatzgrad der Olefine in der ersten Hydroformylierungsstufe wird auf den gewünschten Wert begrenzt, indem man die Reaktionsbedingungen der Hydroformylierung zweckentsprechend verändert. Durch Wahl von niedrigeren Reaktionstemperaturen und/oder Katalysatorkonzentrationen sowie kürzeren Verweilzeiten kann der Olefinumsatz im Reaktor gesenkt werden. Der Umsatzgrad der Olefine im ersten Reaktor wird auf Basis der Menge und der Zusammensetzung des frischen Olefingemisches 2 sowie der Menge und der Zusammensetzung des Hydroformylierungsgemisches 5 bestimmt. Der Gesamtumsatzgrad wird auf Basis der Menge und der Zusammensetzung des frischen Olefingemisches 2 sowie der Menge und der Zusammensetzung der ausgeschleusten Leichtsieder (16 gemäß beiden Figuren) ermittelt. Für die Bestimmung der Olefingehalte in den verschiedenen Stoffströmen kann die gaschromatographische Analyse angewandt werden.

### Katalysatorabtrennung

Die Reaktionsgemische der Hydroformylierung werden, wie zuvor erwähnt, zweckmäßig zunächst vom Katalysator befreit, wiederum in an sich bekannter Weise. Wenn ein Kobaltkatalysator verwendet wurde, kann dies durch Druckentlastung, Abtrennung der wäßrigen Katalysatorphase, Oxidation der im Hydroformylierungsgemisch verbliebenen Kobaltcarbonylverbindungen mit Luft oder Sauerstoff und Auswaschen der entstandenen Kobaltverbindungen mit Wasser oder wäßriger Säure geschehen. Entkobaltungsverfahren sind gut bekannt, siehe z.B. J.Falbe, a.a.O. 164, 165 (BASF-Process), Kirk-Othmer, a.a.O., sowie EP-0 850 905 A1. Wenn eine Rhodiumverbindung als Hydroformylierungskatalysator diente, kann man sie mittels Dünnschichtverdampfung als Destillationsrückstand vom Hydroformylierungsgemisch abtrennen.

Wenn die bevorzugten Kobalkatalysatoren verwendet wurden, enthalten die vom Katalysator befreiten Reaktionsgemische der ersten Hydroformylierungsstufe, je nach dem Umsatzgrad, im allgemeinen 8-45 Gew.-%. meistens 15-35 Gew.-% Leichtsieder mit niedrigerem Siedepunkt als die Aldehyde, hauptsächlich Olefine, daneben die entsprechenden gesättigten Kohlenwasserstoffe sowie Wasser und Methanol; weiterhin 30-80 Gew.-% Aldehyde, 5-30 Gew.-% Alkohole, bis zu 10 Gew.-% Formiate der Alkohole und 0,5-5 Gew.-% Hochsieder mit höherem Siedepunkt als die Alkohole.

In den Reaktionsgemischen der zweiten Hydroformylierungsstufe sind dann im allgemeinen 10-40 Gew.-%, meistens 15-30 Gew.-% Leichtsieder. darunter weniger Olefine und viel gesättigte Kohlenwasserstoffe sowie Wasser und Methanol, weiterhin 30-70 Gew.-% Aldehyde, 5-40 Gew.-% Alkohole, bis zu 10 Gew.-% Formiate dieser Alkohole und 3-12 Gew.-% Hochsieder mit höherem Siedepunkt als die Alkohole enthalten.

Bei Verwendung von Rhodiumkatalysatoren enthalten die Reaktionsgemische deutlich weniger Paraffine und Formiate.

### Selektive Hydrierung

Die selektive Hydrierung der zweckmäßig vom Katalysator befreiten Reaktionsgemische aus beiden Hydroformylierungsstufen ist ein wesentliches Merkmal des erfindungsgemäßen Verfahrens. Dadurch werden die Aldehyde und bestimmte Begleitstoffe, darunter Acetale der Aldehyde und Ester der Alkohole und von diesen insbesondere die Formiate, zu den gewünschten Alkoholen hydriert. Dabei werden die nicht umgesetzten Olefine nicht oder praktisch nicht hydriert, so daß hohe Ausbeuten in bezug auf die eingesetzten Olefingemische erzielt werden. Weniger als 5 % der eingesetzten Olefine gehen durch Hydrierung zu den gesättigten Kohlenwasserstoffen verloren.

Eine selektive Hydrierung von Hydroformylierungsgemischen ist Gegenstand der gleichzeitig anhängigen Patentanmeldung 198 42 370.5 (O.Z. 5356). Die Reaktionsgemische der Hydroformylierung werden danach mittels Wasserstoff bei erhöhter Temperatur und unter erhöhtem Druck an einem Trägerkatalysator hydriert, der als aktive Komponenten Kupfer, Nickel und Chrom enthält.

Bevorzugte Katalysatoren dieser Art sind Trägerkatalysatoren, die als aktive Komponenten Kupfer und Nickel in Konzentrationen von jeweils 0,3 bis 15 Gew.-%. Chrom in einer Konzentration von 0,05 bis 3,5 Gew.-% sowie eine Alkalimetallkomponente in einer Konzentration von 0,01 bis 1,6 Gew.-%, vorteilhaft von 0,02-1,2 Gew.-% enthalten, jeweils bezogen auf den Trägerkatalysator. Ein anderer vorteilhafter Trägerkatalysator enthält Kupfer, Nickel und Chrom in den angegebenen Mengen, aber keine Alkalimetallkomponente. Geeignete Trägerstoffe sind insbesondere Siliciumdioxid und Aluminiumoxid. Die Mengenangaben beziehen sich auf den wie später beschrieben hergestellten, noch nicht reduzierten Katalysator.

Bei der Hydrierung werden die Aldehyde in den Reaktionsgemischen beider Hydroformylierungsstufen jeweils bei Umsätzen über 98% in einer Selektivität von über 99% bei nur einer Hydrierstufe zu den entsprechenden Alkoholen hydriert. Die Ester und die Acetale werden ebenfalls in die gewünschten Alkohole umgewandelt. Die im Gemisch enthaltenen Ausgangsolefine bleiben überraschenderweise weit überwiegend unverändert, obwohl die bevorzugten Trägerkatalysatoren unter vergleichbaren Bedingungen auch die olefinische Doppelbindung im 2-Ethylhex-2-enal praktisch quantitativ hydrieren (EP 0 326 674 A2). Die Hydrierung kann im Niederdruckbereich von unter 30 bar und mit hohen Raum-Zeit-Ausbeuten durchgeführt werden.

Die genannten Katalysatorkomponenten können homogen in den Poren eines Trägermaterials verteilt oder in dessen Randzonen angereichert sein. Im ersteren Fall setzt man eine wässerige Lösung an, die die Komponenten in Form von Metallsalzen enthält und deren Volumen zweckmäßig ungefähr dem 0,8-fachen des Porenvolumens des Trägermaterials entspricht. Als Kupfer-, Nickel- und Chromsalze als Katalysatorvorläufer verwendet man vorteilhaft solche, die beim Erhitzen in Oxide übergehen, wie Nitrate und Acetate. Wenn der Katalysator eine Alkalimetallkomponente enthalten soll, kann diese zusammen mit Chrom in Form von Alkalichromat oder -dichromat, insbesondere als Natriumchromat oder -dichromat eingebracht werden. Die Konzentration der Metallsalze in der Lösung hängt von der gewünschten Konzentration der jeweiligen Komponente im fertigen Katalysator ab. Die Metallsalzlösung wird dann auf das in einer Dragiertrommel befindliche, nicht vorerhitzte Trägermaterial aufgesprüht und dringt in dessen Poren ein. Danach wird der Katalysator getrocknet.

Ist ein Katalysator mit Komponenten erwünscht, die in den Randzonen eines porösen oder eines mehr oder minder porenfreien Trägermaterials angereichert sind, so kann man die Metallsalzlösung auf das vorerhitzte Trägermaterial aufsprühen und das Trägermaterial während des Aufsprühens weiter erhitzen, so daß das Wasser verdampft und die Katalysatorkomponenten im wesentlichen auf der Oberfläche des Trägermaterials fixiert werden.

Nach dem Aufbringen der Katalysatorkomponenten werden die Katalysatoren beider genannten Typen calciniert. d.h. je nach dem Katalysatorvorläufer auf Temperaturen von 200-400°C erhitzt, wodurch die Katalysatorvorläufer in den oxidischen Zustand überführt werden. Danach wird der Katalysator mit Wasserstoff bei den genannten Hydriertemperaturen reduziert. Die Reduktion kann gleich nach der Herstellung des Katalysators oder zweckmäßig erst in dem Hydrierreaktor erfolgen.

Die Katalysatoren werden vorteilhaft in einer Form eingesetzt, in der sie einen geringen Strömungswiderstand bieten, z.B. in Form von Granalien, Pellets oder Formkörpern, wie Tabletten, Zylindern oder Ringen. Sie werden zweckmäßig vor ihrem Einsatz durch Erhitzen im Wasserstoffstrom, z.B. auf die genannten Hydriertemperaturen, aktiviert, sofern sie nicht im Reaktor reduziert wurden.

Die Hydrierung kann kontinuierlich oder diskontinuierlich und sowohl in der Gasphase als auch in flüssiger Phase durchgeführt werden. Die Hydrierung in flüssiger Phase wird bevorzugt, weil das Gasphasenverfahren wegen der notwendigen Kreisführung großer Gasvolumina einen höheren Energieaufwand erfordert. Hinzu kommt, daß die Verdampfung der Aldehyde mit steigender C-Zahl mehr und mehr Energie erfordert und zudem die Eduktbeladung des Reduktionsgases abnimmt, so daß ein Gasphasenverfahren bei Aldehyden mit einer C-Zahl größer als etwa 8 das Gesamtverfahren kaum noch wirtschaftlich durchführbar macht.

Für die Flüssigphasenhydrierung können unterschiedliche Verfahrensvarianten gewählt werden. Sie kann adiabatisch oder praktisch isotherm, d.h. mit einem Temperaturanstieg von <10°C, ein- oder zweistufig durchgeführt werden. Im letzteren Fall kann man beide Reaktoren, zweckmäßig Rohrreaktoren, adiabatisch oder praktisch isotherm oder den einen adiabatisch und den anderen praktisch isotherm betreiben. Weiterhin ist es möglich, die Hydroformylierungsgemische im geraden Durchgang oder mit Produktrückführung zu hydrieren. Die Reaktoren können als Gleichstromreaktoren mit Rieselbett (trickle flow) oder bevorzugt mit hohen Flüssigkeitsbelastungen (pulse flow) betrieben werden. Im Interesse einer hohen Raum-Zeit-Ausbeute werden die Reaktoren vorzugsweise mit hohen Flüssigkeitsbelastungen von 5-100 m³, insbesondere von 15-50 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 0.1 und 10 h⁻¹, vorzugsweise zwischen 0.5 und 5 h⁻¹ annehmen.

Die Flüssigphasenhydrierung wird im allgemeinen unter einem Gesamtdruck von 5 bis 30 bar, insbesondere zwischen 15 und 25 bar durchgeführt. Die Hydrierung in der Gasphase kann auch bei niedrigeren Drükken durchgeführt werden, mit entsprechend großen Gasvolumina. Die Reaktionstemperaturen liegen bei Hydrierungen in flüssiger oder gasförmiger Phase in der Regel zwischen 120 und 220°C, insbesondere zwischen 140 und 180°C.

### Auftrennung der Hydrierungsgemische durch Destillation

Nach der Hydrierung werden die Reaktionsgemische in an sich bekannter Weise durch Destillation aufgearbeitet. Als Kopfprodukt werden Leichtsieder abgetrennt, die überwiegend Olefine und daneben gesättigte Kohlenwasserstoffe enthalten. Gemäß Figur 1 sind die Leichtsieder aus beiden Hydroformylierungsstufen Edukt für die zweite Hydroformylierungsstufe. Bei dieser Verfahrensvariante wird allerdings ein Teil dieser Leichtsieder ausgeschleust, damit die Konzentration der inerten, durch Hydrierung der Olefine in den Hydroformylierungsstufen entstandenen gesättigten Kohlenwasserstoffe auf einer akzeptablen Höhe von höchstens 60% gehalten wird. Bei der Verfahrenvariante nach Figur 2 werden die gesamten Leichtsieder aus der ersten Destillation in die zweite Hydroformylierungsstufe geführt, und die gesättigten Kohlenwasserstoffe werden als vergleichsweise kleine Leichtsiederfraktion der zweiten Destillation ausgeschleust.

Die Destillation der Hydrierungsgemische wird in der Regel bei vermindertem Druck, z.B. bei einem absoluten Druck von 400-900 mbar, durchgeführt. Der rohe Alkohol, der bei der Destillation als Sumpfprodukt anfällt, kann in üblicher Weise durch Destillation auf reinen Alkohol aufgearbeitet werden.

Die folgenden Beispiele sollen die Erfindung weiter erläutern, nicht aber ihren Anwendungsbereich beschränken, der sich aus den Patentansprüchen ergibt.

### Beispiel 1 (Vergleichsbeispiel)

Nonanole durch einstufige Hydroformylierung von Di-n-buten In einem 5l-Hochdruckautoklaven, der mit einem Rührer und elektrischer Beheizung versehen war, wurden 2000 g Di-n-buten (Zusammensetzung in Tabelle 1, Spalte 2) in Gegenwart eines Kobaltkatalysators bei 185°C und konstant gehaltenem Synthesegasdruck von 280 bar hydroformyliert. Das Synthesegas enthielt 50 Vol.-% CO und 50 Vol.-% H₂.

Zur Herstellung der als Katalysator dienenden Kobalthydridocarbonyle, z.B. HCo(CO)₄, wurde als Katalysator-Vorläufer eine wäßrige Kobaltacetat-Lösung mit 1 Gew.-% Co verwendet. Die Kobaltacetat-Lösung wurde unter Rühren 7 h bei 170°C und 280 bar mit Synthesegas behandelt. Nach Abkühlen auf Raumtemperatur und Entspannen wurden die gebildeten Kobaltcarbonyle durch Extraktion mit Di-n-buten in die organische Phase überführt. Nach Abtrennen der wäßrigen Phase wurde das mit Kobaltcarbonylen beladene Di-n-buten mit einem Gehalt von 0,021 Gew.-% Co (gerechnet als Metall) unter den oben genannten Reaktionsbedingungen 3 Stunden lang hydroformyliert.

Nach Abkühlung auf Raumtemperatur wurde das Reaktionsgemisch entspannt aus dem Autoklaven entleert und durch Behandlung mit 5 %-iger Essigsäure und Luft bei 80°C vom Kobaltkatalysator befreit. Es wurden 2487 g entkobaltetes Hydroformylierungsgemisch erhalten, die mittels Gaschromatographie analysiert wurden. Die Ergebnisse sind in Tabelle 2, Spalte 2 aufgeführt. Danach wurde ein Di-n-buten-Umsatz von 92.3% bei einer Wertprodukt-Selektivität von 87.9% erzielt, entsprechend einer Wertproduktausbeute von 81,1%, bezogen auf eingesetztes Di-n-buten. Als Wertprodukte wurden C₉-Aldehyde, C₉-Alkohole und (Iso)nonylformiate betrachtet.

### Beispiel 2

Nonanole durch zweistufige Hydroformylierung - 1. Stufe In einem 5l-Hochdruckautoklaven, der mit einem Rührer und elektrischer Beheizung versehen war, wurden 2000 g Di-n-buten (Zusammensetzung in Tabelle 1, Spalte 2) in Gegenwart eines Kobaltkatalysators bei 170°C und einem konstant gehaltenen Synthesegasdruck von 280 bar hydroformyliert. Das Synthesegas enthielt wiederum 50 Vol.-% CO und 50 Vol.-% H₂.

Der Kobaltkatalysator wurde wie in Beispiel 1 hergestellt und in das Di-n-buten überführt. Die Konzentration des Katalysators betrug 0,019 Gew.-% Co, bezogen auf das Di-n-buten. Das mit Kobaltcarbonylen beladene Di-n-buten wurde unter den oben genannten Reaktionsbedingungen 2 Stunden lang hydroformyliert. Das Hydroformylierungsgemisch wurde wie in Beispiel 1 vom Kobaltkatalysator befreit.

Dieser Hydroformylierungsansatz wurde unter denselben Bedingungen dreimal wiederholt. Die Hydroformylierungsgemische wurden nach Entfernung des Kobalt-Katalysators vereinigt. Es wurden 9412 g Reaktionsgemisch erhalten, das nach GC-Analyse die in der Tabelle 2, Spalte 3 angegebene Zusammensetzung hatte. Danach wurde ein Di-n-buten-Umsatz von 67,6% bei einer Wertprodukt-Selektivität von 94.5% erzielt, entsprechend einer Wertproduktausbeute von 63,9%, bezogen auf eingesetztes Di-n-buten. Als Wertprodukte wurden wieder C₉-Aldehyde, C₉-Alkohole und (Iso)nonylformiate betrachtet.

### Beispiel 3

Nonanole durch zweistufige Hydroformylierung - 2. Stufe 7500 g des Reaktionsgemisches aus Beispiel 2 wurden selektiv unter Erhalt der Olefine zum Wertprodukt C₉-Alkohol hydriert. Die Hydrierung wurde diskontinuierlich in einem 20 l-Autoklaven bei 175°C und 20 bar H₂-Druck in der Flüssigphase in Gegenwart eines Trägerkatalysators mit 12,1 Gew.-% Cu, 3,0 Gew.-% Ni und 2,5 Gew-% Cr auf Aluminiumoxid als Trägermaterial durchgeführt. Anschließend wurden aus dem Hydrierungsgemisch die nicht umgesetzten Olefine als Leichtsieder von den Wertprodukten und den Schwersiedern abdestilliert.

Die Leichtsiederfraktion enthielt nach GC-Analyse neben 98,5 Gew.-% Kohlenwasserstoffen, davon 87,9 Gew.-% C₈-Olefine, rund 1,5 Gew.-% Methanol, das durch Hydrierung von (Iso)nonylformiaten entstanden war. Die Isomerenverteilung der Isomeren im C₈-Isomerengemisch ist in Tabelle 1. Spalte 3 aufgeführt. Im Vergleich zum frischen Di-n-buten mit 23 Gew.-% Dimethylhexenen enthielt dieses C₈-Isomerengemisch mit 44 Gew.-% Dimethylhexenen deutlich größere Mengen an diesen reaktionsträgeren C₈-Isomeren.

2000g dieses mit Dimethylhexenen angereicherten und 10,6 Gew.-% C₈-Paraffine enthaltenden C₈-Olefinisomerengemisches wurden in einem 5 l-Autoklaven bei 185°C und einem Synthesegasdruck von 280 bar in Gegenwart eines Kobaltkatalysators auf die in Beispiel 1 beschriebene Weise hydroformyliert. Es wurde wiederum ein Synthesegas mit 50 Vol.-% CO und 50 Vol.-% H₂ verwendet. Bei einem Kobaltgehalt von 0,031 Gew.-%. bezogen auf das C₈-Olefingemisch, wurde dieses Gemisch 3 Stunden lang bei einem konstant gehaltenen Synthesegasdruck hydroformyliert.

Das Hydroformylierungsgemisch wurde entspannt und von Kobaltkatalysator befreit, wie in Beispiel 1 beschrieben. Man erhielt 2438 g eines entkobalteten Hydroformylierungsgemisches, dessen durch GC-Analyse Zusammensetzung in Tabelle 2, Spalte 4 wiedergegeben ist. Danach wurde ein C₈-Olefinumsatz von 91,3% bei einer Wertprodukt-Selektivität von 83,0% erzielt, entsprechend einer Wertproduktausbeute von 75,8%, bezogen auf eingesetztes Di-n-buten. Als Wertprodukte wurden C₉-Aldehyde, C₉-Alkohole und (Iso)nonylformiate betrachtet.

Faßt man das Beispiel 2 als erste Hydroformylierungsstufe und das Beispiel 3 als zweite Hydroformylierungsstufe des erfindungsgemäßen Verfahrens zusammen, so wurde nach beiden Stufen ein Gesamtolefinumsatz von 97,1 % bei einer Wertproduktselektivität von 91,5%, entsprechend einer Gesamtwertproduktausbeute von 88,8%, bezogen auf eingesetztes Olefingemisch, erzielt.

Gegenüber der einstufigen Hydroformylierung nach Beispiel 1 ergibt sich also eine Steigerung der Wertproduktausbeute um rund 8 Prozentpunkte.

**Tabelle 1**

| Verteilung der C₈-Isomeren im Einsatz-Olefingemisch | | |
|---|---|---|
| 1 | 2 | 3 |
| C₈-Isomere | Di-n-buten Edukt Beispiele 1 und 2 (Gew.-%) | C₃-Olefingemisch Edukt Beispiel 3 (Gew.-%) |
| Dimethylhexene | 23 | 44 |
| 3-Methylheptene | 62 | 51 |
| n-Octene | 15 | 5 |

**Tabelle 2**

| Zusammensetzung von Hydroformylierungsgemischen | | | |
|---|---|---|---|
| 1 | 2 | 3 | 4 |
| Zusammensetzung nach GC-Analyse | Beispiel 1 (Gew.-%) | Beispiel 2 (Gew.-%) | Beispiel 3 (Gew.-%) |
| C₈-Olefine | 6,2 | 27,4 | 7,0 |
| C₈-Paraffine | 3,3 | 2,4 | 4,5 |
| Isononanale | 55,1 | 48,7 | 51,2 |
| Ester/Isononylformiate | 4,5 | 2,0 | 8,8 |
| Isononanole | 23,9 | 18,5 | 18,8 |
| Rückstand | 7,0 | 1,0 | 9,8 |

## Patentansprüche

1. Verfahren zur Herstellung höherer Oxo-Alkohole aus Gemischen isomerer Olefine mit 5 bis 24 Kohlenstoffatomen durch zweistufige Hydroformylierung in Gegenwart eines Kobalt- oder Rhodium-Katalysators bei erhöhter Temperatur und unter erhöhtem Druck,
**dadurch gekennzeichnet,**
**dass** man das Reaktionsgemisch der ersten Hydroformylierungsstufe (1), bei erhöhter Temperatur und unter erhöhtem Druck an einem Trägerkatalysator, der als aktive Komponenten Kupfer, Nickel und Chrom enthält, selektiv hydriert (9), das Hydrierungsgemisch (10) in einer Destillation (11) in rohen Alkohol und überwiegend aus Olefinen bestehende Leichtsieder trennt, diese in die zweite Hydroformylierungsstufe (13) führt, das Reaktionsgemisch der zweiten Hydroformylierungsstufe wiederum selektiv hydriert (9, 22), das Hydrierungsgemisch in einer Destillation (11, 24) in rohen Alkohol und Leichtsieder trennt, den rohen Alkohol (21) durch Destillation auf reinen Alkohol aufarbeitet und zumindest einen Teil der Leichtsieder zur Ausschleusung gesättigter Kohlenwasserstoffe abzieht.

2. Verfahren nach Anspruch 1,
**dadurch gekennzeichnet,**
**dass** man die entspannten Reaktionsgemische aus beiden Hydroformylierungsstufen vor der selektiven Hydrierung vom Hydroformylierungskatalysator (7 und 19) befreit.

3. Verfahren nach Anspruch 1 oder 2,
**dadurch gekennzeichnet,**
**dass** man C₈-, C₉-, C₁₂- oder C₁₆-Olefingemische als Edukte für die Hydroformylierung einsetzt.

4. Verfahren nach einem der Ansprüche 1 bis 3,
**dadurch gekennzeichnet,**
**dass** man in beiden Hydroformylierungsstufen mit Kobaltkatalysatoren arbeitet.

5. Verfahren nach einem der Ansprüche 1 bis 4,
**dadurch gekennzeichnet,**
**dass** nur eine selektive Hydrierung vorgesehen wird, in denen beide gegebenenfalls entkatalysierte Reaktionsgemische der Hydroformylierungsstufen (1) und (13) selektiv hydriert werden, und nur eine Destillation (11) in der das Hydrierungsgemisch getrennt wird.

6. Verfahren nach Anspruch 5,
**dadurch gekennzeichnet,**
**dass** ein Teil der Leichtsieder aus der Destillation (11, 24) zur Ausschleusung von Paraffinen abgezogen wird.

7. Verfahren nach einem der Ansprüche 1 bis 4,
**dadurch gekennzeichnet,**
**dass** das gegebenenfalls entkatalysierte Reaktionsgemisch der zweiten Hydroformylierungsstufe (13) in einer zweiten selektiven Hydrierung (22) hydriert und das Hydrierungsgemisch in einer zweiten Destillation (24) getrennt wird.

8. Verfahren nach Anspruch 7,
**dadurch gekennzeichnet,**
**dass** die Leichtsieder aus der zweiten Destillation zur Ausschleusung von Paraffinen abgezogen werden.

9. Verfahren nach einem der Ansprüche 1 bis 8,
**dadurch gekennzeichnet,**
**dass** man einen Trägerkatalysator verwendet, der als aktive Komponenten Kupfer und Nickel in Konzentrationen von jeweils 0,3 bis 15 Gew.-%, Chrom in einer Konzentration von 0,05 bis 3,5 Gew.-% und eine Alkalimetallkomponente in einer Konzentration von 0,01 bis 1,6 Gew.-%, jeweils bezogen auf den Trägerkatalysator, enthält.

10. Verfahren nach Anspruch 9,
**dadurch gekennzeichnet,**
**dass** die Konzentration der Alkalimetallkomplexe 0,2 bis 1,2 Gew.-% beträgt.

11. Verfahren nach Anspruch 9,
**dadurch gekennzeichnet,**
**dass** der Trägerkatalysator keine Alkalimetallkomponente enthält.

12. Verfahren nach einem der Ansprüche 1 bis 11,
**dadurch gekennzeichnet,**
**dass** das Trägermaterial des Katalysators Siliciumdioxid oder Aluminiumoxid ist.

13. Verfahren nach einem der Ansprüche 1 bis 12,
**dadurch gekennzeichnet,**
**dass** man die Hydrierung kontinuierlich oder diskontinuierlich in flüssiger Phase durchführt.

14. Verfahren nach einem der Ansprüche 1 bis 13,
**dadurch gekennzeichnet,**
**dass** man die Hydrierung in flüssiger Phase unter einem Gesamtdruck von 5 bis 30 bar durchführt.

15. Verfahren nach Anspruch 14,
**dadurch gekennzeichnet,**
**dass** der Gesamtdruck 15 bis 25 bar beträgt.

16. Verfahren nach einem der Ansprüche 1 bis 15, **dadurch gekennzeichnet, dass** man die Hydrierung bei 120 bis 220°C °C durchführt.

17. Verfahren nach Anspruch 16, **dadurch gekennzeichnet, dass** die Temperatur 140 bis 180 °C beträgt.

18. Verfahren nach einem der Ansprüche 1 bis 17, **dadurch gekennzeichnet, dass** man die Hydrierung in flüssiger Phase und mit Flüssigkeitsbelastungen von 5-100 m³ pro m² Querschnitt des leeren Reaktors und Stunde durchführt.

19. Verfahren nach Anspruch 18, **dadurch gekennzeichnet, dass** die Flüssigkeitsbelastung 15-30 m³ pro m² Querschnitt des leeren Reaktors und Stunde beträgt.

## Claims

1. A process for preparing higher oxo alcohols from mixtures of isomeric olefins having from 5 to 24 carbon atoms by two-stage hydroformylation in the presence of a cobalt catalyst or rhodium catalyst at elevated temperature and at elevated pressure, **characterized in that** the reaction mixture from the first hydroformylation stage (1) is selectively hydrogenated (9) at elevated temperature and at elevated pressure on a supported catalyst which comprises, as active components, copper, nickel and chromium, the hydrogenation mixture (10) is separated by distillation (11) into crude alcohol and low-boilers predominantly consisting of olefins, these low-boilers are passed to the second hydroformylation stage (13), the reaction mixture from the second hydroformylation stage is again selectively hydrogenated (9, 22), the hydrogenation mixture is separated in a distillation (11, 24) into crude alcohol and low-boilers, the crude alcohol (21) is worked up by distillation to pure alcohol and at least some of the low-boilers are taken off to discharge saturated hydrocarbons.

2. A process according to claim 1, **characterized in that** the expanded reaction mixtures from both hydroformylation stages are freed from the hydroformylation catalyst (7 and 19) prior to the selective hydrogenation.

3. A process according to claim 1 or 2, **characterized in that** C₈, C₉, C₁₂ or C₁₆ olefin mixtures are used as starting materials for the hydroformylation.

4. A process according to any one of claims 1 to 3, **characterized in that** cobalt catalysts are employed in both hydroformylation stages.

5. A process according to any one of claims 1 to 4, **characterized in that** only one selective hydrogenation is provided in which the reaction mixtures from both hydroformylation stages (1) and (13), catalyst-depleted if desired, are selectively hydrogenated and only one distillation (11) in which the hydrogenation mixture is separated is provided.

6. A process according to claim 5, **characterized in that** some of the low-boilers from the distillation (11, 24) are taken off to discharge paraffins.

7. A process according to any one of claims 1 to 4, **characterized in that** the reaction mixture from the second hydroformylation stage (13), catalyst-depleted if desired, is hydrogenated in a second selective hydrogenation (22) and the hydrogenation mixture is separated in a second distillation (24).

8. A process according to claim 7, **characterized in that** the low-boilers from the second distillation are taken off to discharge paraffins.

9. A process according to any one of claims 1 to 8, **characterized in that** use is made of a supported catalyst which comprises, as active components, copper and nickel at concentrations in each case of from 0.3 to 15% by weight, chromium at a concentration of from 0.05 to 3.5% by weight and an alkali metal component at a concentration of from 0.01 to 1.6% by weight, in each case based on the supported catalyst.

10. A process according to claim 9, **characterized in that** the concentration of the alkali metal complex is from 0.2 to 1.2% by weight.

11. A process according to claim 9, **characterized in that** the supported catalyst does not contain an alkali metal component.

12. A process according to any one of claims 1 to 11, **characterized in that** the catalyst support material is silicon dioxide or aluminium oxide.

13. A process according to any one of claims 1 to 12, **characterized in that** the hydrogenation is carried out continuously or batchwise in the liquid phase.

14. A process according to any one of claims 1 to 13, **characterized in that** the hydrogenation is carried out in the liquid phase under an overall pressure of from 5 to 30 bar.

15. A process according to claim 14, **characterized in that** the overall pressure is from 15 to 25 bar.

16. A process according to any one of claims 1 to 15, **characterized in that** the hydrogenation is carried out at from 120 to 220°C.

17. A process according to claim 16, **characterized in that** the temperature is from 140 to 180°C.

18. A process according to any one of claims 1 to 17, **characterized in that** the hydrogenation is carried out in the liquid phase and with liquid flow rates of 5-100 m³ per m² of cross section of the empty reactor and hour.

19. A process according to claim 18, **characterized in that** the liquid flow rate is 15-50 m³ per m² of cross section of the empty reactor and hour.

## Revendications

1. Procédé de production d'oxoalcools supérieurs à partir de mélanges d'oléfines isomères ayant de 5 à 24 atomes de carbone par hydroformylation en deux étapes en présence d'un catalyseur au cobalt ou au rhodium à une température accrue et sous pression accrue,
**caractérisé en ce qu'**
on hydrogènise (9) sélectivement le mélange réactionnel du premier stade de l'hydroformylation (1) à température accrue et sous pression accrue sur un catalyseur sur support, qui contient comme composant actif du cuivra, du nickel et du chrome, on sépare le mélange d'hydrogénation (10) dans une distillation (11) en un alcool brut et principalement un produit à bas point d'ébullition consistant principalement en des oléfines, on conduit ceux-ci dans le deuxième stade d'hydroformylation (13), on hydrogènise à nouveau sélectivement le mélange réactionnel du deuxième stade d'hydroformylation (9, 22), on sépare le mélange d'hydrogénation dans une distillation (11, 24) en alcool brut et en produit à bas point d'ébullition, on purifie l'alcool brut (21) par distillation en alcool pur et on soutire au moins une partie du produit à bas point d'ébullition en vue d'évacuer les hydrocarbures saturés.

2. Procédé selon la revendication 1,
**caractérisé en ce qu'**
on débarrasse les mélanges réactionnels détendus provenant des deux étapes d'hydroformylation avant l'hydrogénation sélective, du catalyseur d'hydroformylation (7 et 19).

3. Procédé selon la revendication 1 ou 2,
**caractérisé en ce qu'**
on met en oeuvre des mélanges d'oléfines en C₈, C₉, C₁₂ ou C₁₆ comme éduits pour l'hydroformylation.

4. Procédé selon l'une des revendications 1 à 3,
**caractérisé en ce qu'**
on opère dans les deux stades d'hydroformylation avec des catalyseurs au cobalt.

5. Procédé selon l'une des revendications 1 à 4,
**caractérisé en ce qu'**
on prévoit seulement une hydrogénation sélective dans laquelle les deux mélanges réactionnels décatalysée éventuellement, des stades d'hydroformylation (1) et (13) sont sélectivement hydrogénés et seulement une distillation (11) dans laquelle le mélange d'hydrogénation est séparé.

6. Procédé selon la revendication 5,
**caractérisé en ce qu'**
une partie de la fraction à bas point d'ébullition provenant de la distillation (11, 24) est soutiré en vue de l'évacuation des paraffines.

7. Procédé selon l'une des revendications 1 à 4,
**caractérisé en ce que**
le mélange réactionnel décatalysé éventuellement du deuxième stade d'hydroformylation (13) est hydrogéné dans une deuxième hydrogénation sélective (22) et le mélange d'hydrogénation est séparé dans une deuxième distillation (24).

8. Procédé selon la revendication 7,
**caractérisé en ce que**
la fraction à bas point d'ébullition provenant de la deuxième distillation est soutirée en vue de l'évacuation des paraffines.

9. Procédé selon l'une des revendications 1 à 8,
**caractérisé en ce qu'**
on utilise un catalyseur sur support qui contient comme composants actifs du cuivre et du nickel à des concentrations de respectivement 0,3 à 15 % en poids, du chrome à une concentration de 0,05 à 3,5 % en poids et un composant de métal alcalin à une concentration de 0,01 à 1,6 % en poids, à chaque fois rapporté au catalyseur sur support.

10. Procédé selon la revendication 9,
**caractérisé en ce que**
la concentration des complexes de métal alcalin s'élève à de 0,2 à 1,2 % en poids.

11. Procédé selon la revendication 9,
**caractérisé en ce que**
le catalyseur sur support ne contient aucun composant de métal alcalin.

12. Procédé selon l'une des revendications 1 à 11,
**caractérisé en ce que**
le matériau de support du catalyseur est du dioxyde de silicium ou de l'oxyde d'aluminium.

13. Procédé selon l'une des revendications 1 à 12,
**caractérisé en ce qu'**
on effectue l'hydrogénation en continu ou d'une manière discontinue en phase liquide.

14. Procédé selon l'une des revendications 1 à 13,
**caractérisé en ce qu'**
on effectue l'hydrogénation en phase liquide sous une pression totale allant de 5 à 30 bars.

15. Procédé selon la revendication 14,
**caractérisé en ce que**
la pression totale s'élève de 15 à 25 bars.

16. Procédé selon l'une des revendications 1 à 15,
**caractérisé en ce qu'**
on effectue l'hydrogénation à 120 à 220°C.

17. Procédé selon la revendication 16,
**caractérisé en ce que**
la température s'élève à de 140 à 180°C.

18. Procédé selon l'une des revendications 1 à 17,
**caractérisé en ce qu'**
on effectue l'hydrogénation en phase liquide et avec des contraintes de liquide de 5 à 100 m³ par m² de section du réacteur liquide et par heure.

19. Procédé selon la revendication 18,
**caractérisé en ce que**
la contrainte de liquide s'élève à 15 à 30 m³ par m² de section du réacteur vide et par heure.
